# EUROPEAN PATENT APPLICATION

(11) **EP 4 260 877 A1**
(43) Date de publication de la demande: **18.10.2023**
(21) Numéro de dépôt: 22425016.7
(22) Date de dépôt: 11.04.2022
(51) Int. Cl.: A61L 2/04, A61L 2/07, B65B 55/04, B65B 55/10, B65B 55/14

(54) **EMBALLAGE DE STERILISATION ET PROCEDE DE STERILISATION CORRESPONDANT**

(71) Demandeur: ELIS ITALIA S.p.A., 20098 San Giuliano Milanese (MI) (IT)
(72) Inventeur: Viscardi, Mario Federico, 20124 Milano (MI) (IT); Corbetta, Marco, 20846 Macherio (MB) (IT)
(74) Mandataire: Zanardo, Giovanni

(57) **Abrégé**

Enveloppe de stérilisation destinée à recevoir un objet à stériliser, comprenant une face avant et une face arrière, jointes sur leur périphérie sur trois bords, la face avant comprenant une première partie (2) réalisée en matériau étanche et une deuxième partie (3) réalisée en matériau poreux, de sorte que le volume de l'emballage en regard de la première partie (2) de la face avant puisse être scellé à la jonction entre la première partie (2) et la deuxième partie (2) après stérilisation afin d'être étanche.

## Description

### Domaine technique

L'invention a pour domaine technique la stérilisation, et plus particulièrement la stérilisation par autoclave à destination des salles blanches équipées de sas de transfert au peroxyde d'hydrogène

### Techniques antérieures

La stérilisation des articles pour salle blanche, notamment des accessoires ou tenues, est réalisée par exposition à un rayonnement gamma ou beta, ou par stérilisation thermique en autoclave.

L'exposition à un rayonnement gamma permet d'utiliser des emballages imperméables, mais est particulièrement délicate à gérer, est associée à un cout élevé et réduit drastiquement la durée de vie des articles ainsi stérilisés.

La stérilisation thermique en autoclave à vapeur d'eau oblige à utiliser un emballage avec une partie de la surface perméable à la vapeur (papier, tnt également appelé tissu non tissé, ou autre). Cette modalité de stérilisation est plus sure, peut être gérée localement, et ne réduit pas la durée de vie des articles. La stérilisation par autoclave implique l'utilisation d'une enveloppe comprenant une face plastique imperméable, et une face perméable en papier. L'article à stériliser est introduit dans l'enveloppe puis admis dans l'autoclave. L'enceinte de l'autoclave est ensuite saturée de vapeur d'eau chauffée afin de stériliser tous les articles disposés à l'intérieur. La vapeur d'eau chauffée peut pénétrer dans l'enveloppe à travers la face perméable.

Les salles blanches stériles qui utilisent des articles stérilisés à l'extérieur reçoivent ces articles dans des emballages les maintenant stériles. Les salles blanches ont la nécessité de stériliser l'extérieur de l'emballage des articles stérilisés avant de les introduire dans la zone stérile. Pour réaliser cela, on utilise une atmosphère de peroxyde d'hydrogène. Cette stérilisation de la surface extérieure de l'emballage est réalisée par l'intermédiaire d'un sas de transfert (« pass box » en langue anglaise), ouvert selon deux côtés opposés, disposé dans une cloison entre la salle blanche et l'extérieur et permettant le transfert d'articles entre la salle blanche et l'extérieur. Pour pénétrer en salle blanche, un article est introduit dans le sas de transfert par un côté, une porte disposée de sorte à fermer chaque côté opposé du sas est alors verrouillée puis la solution de peroxyde d'hydrogène est introduite. Le sas de transfert ne peut alors être réouvert qu'après évaporation complète de la solution de peroxyde d'hydrogène. Lorsqu'un article est compris dans un emballage pour stérilisation thermique selon l'état de la technique comprenant une membrane poreuse, et, encore plus, lorsque l'article est perméable, par exemple un article textile, la solution de peroxyde d'hydrogène imprègne la membrane et l'article textile et ne peut s'évaporer. Le sas de transfert demeure alors verrouillé.

En pratique les articles stérilisés avec un processus de stérilisation thermique en autoclave ne peuvent pas être introduits à travers un sas de transfert à peroxyde dans un salle blanche stérile. Pour cette raison, seule la stérilisation à rayons gamma peut être employée avec les inconvénients cités plus haut.

Il existe ainsi un besoin pour un procédé de stérilisation thermique permettant d'admettre un article, notamment un article perméable dans une salle blanche par l'intermédiaire d'un sas de transfert à peroxyde d'hydrogène.

### Exposé de l'invention

L'invention a pour objet un emballage de stérilisation comprenant une face avant et une face arrière, jointes sur leur périphérie sur trois bords. La face avant comprend une première partie réalisée en matériau étanche et une deuxième partie réalisée en matériau poreux, de sorte qu'après stérilisation, le volume de l'emballage en regard de la première partie de la face avant puisse être scellé par soudure thermique à la jonction entre la première partie et la deuxième partie afin d'être étanche.

Le matériau étanche peut être une matière plastique souple, notamment du PPE (acronyme pour « Polyphénylène éther »), du polypropylène, du polyester ou un tout autre matériau plastique souple, le matériau poreux pouvant être notamment du papier.

La face arrière peut comprendre une unique partie en matériau étanche

L'emballage de stérilisation peut comprendre des faces périphériques de sorte à former un parallélépipède, les faces périphériques étant réalisées en matériau étanche.

Une première partie de la face arrière peut être réalisée en matériau étanche, une deuxième partie de la face arrière pouvant être réalisée en matériau poreux.

L'emballage de stérilisation peut comprendre des faces périphériques de sorte à former un parallélépipède, les faces périphériques ou parties des faces périphériques en regard des premières parties, étant réalisées en matériau étanche, les faces périphériques ou parties des faces périphériques en regard des deuxièmes parties étant réalisées en matériau poreux.

L'invention a également pour objet un procédé de stérilisation d'un objet comprenant les étapes suivantes :
- Introduction de l'objet à stériliser dans un emballage tel que décrit ci-dessus par l'intermédiaire du quatrième bord ouvert, l'objet étant disposé de sorte à être totalement compris dans un premier volume de l'emballage correspondant au volume de l'emballage en regard de la première partie de la face avant, et fermeture du quatrième bord ouvert,
- Stérilisation de l'emballage de stérilisation comprenant l'objet à stériliser dans un autoclave, l'objet à stériliser étant stérilisé du fait de la présence d'au moins une partie en matériau poreux dans l'emballage, permettant les échanges gazeux, notamment de vapeur, avec l'atmosphère à l'intérieur de l'autoclave,
- Retrait de l'emballage de stérilisation comprenant l'objet stérilisé de l'autoclave,
- Scellement de l'emballage de stérilisation comprenant l'objet stérilisé par soudure thermique à la jonction entre la première partie et la deuxième partie de la face avant de sorte que l'objet stérilisé à l'intérieur soit scellé dans le premier volume de l'emballage entièrement en matériau étanche, et
- Découpe et élimination du deuxième volume de l'emballage de stérilisation.

### Brève description des dessins

D'autres buts, caractéristiques et avantages de l'invention apparaîtront à la lecture de la description suivante, donnée uniquement à titre d'exemple non limitatif et faite en référence aux dessins annexés sur lesquels :
- la figure 1 est une vue de face d'un premier mode de réalisation d'un emballage de stérilisation selon l'invention,
- la figure 2 est une vue en coupe longitudinale du premier mode de réalisation d'un emballage de stérilisation selon l'invention,
- la figure 3 est une vue en coupe longitudinale d'un deuxième mode de réalisation d'un emballage de stérilisation selon l'invention,
- la figure 4 est une vue en coupe longitudinale d'un troisième mode de réalisation d'un emballage de stérilisation selon l'invention,
- la figure 5 est une vue en coupe longitudinale d'un quatrième mode de réalisation d'un emballage de stérilisation selon l'invention,
- la figure 6 illustre les principales étapes d'un procédé de stérilisation selon l'invention,
- la figure 7 illustre le résultat du scellement d'un emballage selon le premier mode de réalisation, et
- la figure 8 illustre le résultat du scellement d'un emballage selon le deuxième mode de réalisation .

### Description détaillée

Afin de résoudre le problème technique de l'état de l'art présenté plus avant, un nouvel emballage et un nouveau procédé de stérilisation ont été développés.

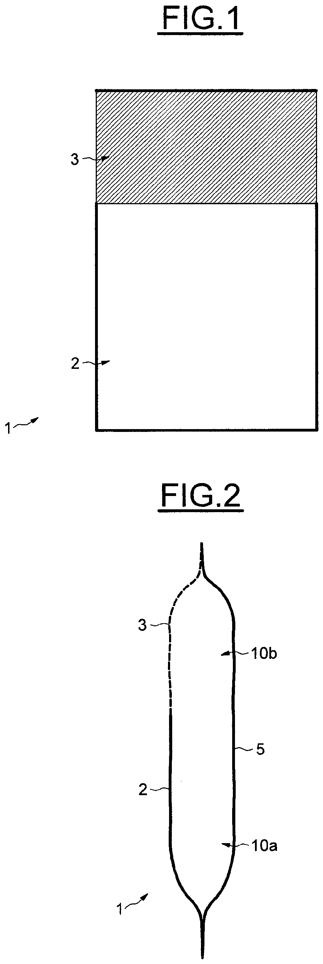

La figure 1 illustre un emballage de stérilisation 1 selon un premier mode de réalisation de l'invention, qui comprend une face avant et une face arrière, opposées et jointes sur leur périphérie sur trois de leurs bords. Le quatrième bord comprend un moyen de fermeture, ou est fermé, notamment par soudure thermique.

La face avant comprend une première partie 2 réalisée en matériau étanche et une deuxième partie 3 réalisée en matériau poreux. Dans un premier mode de réalisation, la face arrière comprend une unique partie 5 en matériau étanche.

Le matériau étanche est de préférence une matière plastique souple, notamment du PPE (acronyme pour « Polyphénylène éther »). Le matériau poreux est notamment du papier.

La figure 2 illustre une vue en coupe de l'emballage selon le premier mode de réalisation.

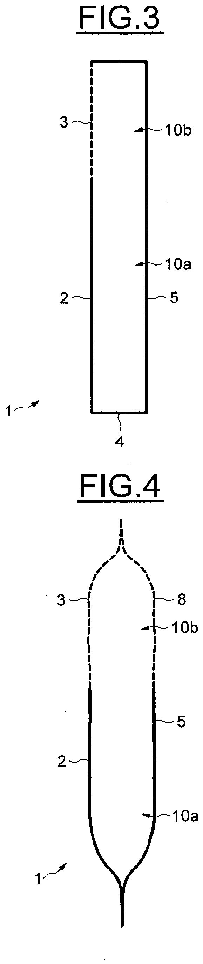

Dans un deuxième mode de réalisation, illustré par la figure 3, l'emballage de stérilisation comprend une face avant similaire à celle de la figure 1. L'emballage de stérilisation selon le deuxième mode de réalisation diffère du premier mode de réalisation du fait qu'il présente une épaisseur réalisée par exemple par des faces périphériques 4 de sorte à former un parallélépipède. Les faces périphériques 4 sont réalisées en matériau étanche et jointes aux faces avant et arrière de l'emballage. La face arrière comprenant une unique partie 5 est illustrée.

Dans un troisième mode de réalisation de l'emballage, dont la figure 4 illustre une vue en coupe, la face avant comprend une première partie 2 réalisée en matériau étanche et une deuxième partie 3 réalisée en matériau poreux. La face arrière comprend une première partie 6 réalisée en matériau étanche et une deuxième partie 8 réalisée en matériau poreux. On note qu'un premier volume 10a, en matériau étanche, est défini entre la première partie 2 de la face avant et la première partie 6 de la face arrière, un deuxième volume 10b, en matériau poreux, étant défini entre la deuxième partie 3 de la face avant et la deuxième partie 8 de la face arrière.

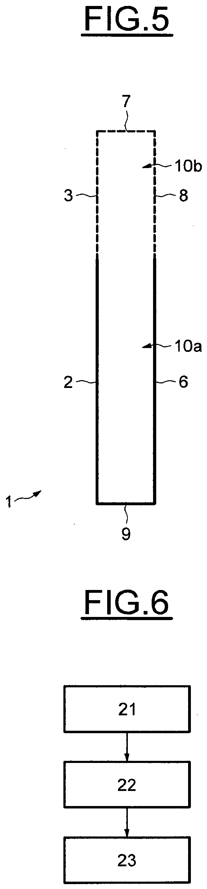

Dans un quatrième mode de réalisation, illustré par la figure 5, l'emballage de stérilisation 1 comprend un premier volume 10a en matériau étanche et un deuxième volume 10b en matériau poreux.

Le premier volume 10a de l'emballage de stérilisation 1 comprend la première partie 2 de la face avant, la première partie 6 de la face arrière et les faces périphériques ou parties des faces périphériques 9 en regard des premières parties 2,6, l'ensemble étant réalisé en matériau étanche.

Le deuxième volume 10b de l'emballage de stérilisation 1 comprend la deuxième partie 3 de la face avant, la deuxième partie 8 de la face arrière et les faces périphériques ou parties des faces périphériques 7 en regard des deuxièmes parties 3,8, tout ou partie de ce deuxième volume 10b étant réalisé en matériau poreux.

L'attention du lecteur est attirée sur le fait que, comme pour le premier et le troisième modes de réalisation, l'emballage de stérilisation 1 peut ne pas présenter d'épaisseur, la face avant et la face arrière étant alors directement jointes sur trois bords en faisant l'impasse sur les faces périphériques. La face avant comprend alors une première partie 2 et une deuxième partie 3, la face arrière comprenant également une première partie 6 et une deuxième partie 8. La face avant et la face arrière sont jointes de sorte que la première partie 2 de la face avant soit directement en regard de la première partie 6 de la face arrière.

Sur les figures 2 à 5, on illustre le premier volume 10a correspondant au volume de l'emballage en regard de la première partie 2 de la face avant, et le deuxième volume 10b en regard de la deuxième partie 3 de la face avant.

Indépendamment du mode de réalisation, l'emballage de stérilisation 1 peut prendre toutes formes et toutes dimensions, dès lors que l'objet à stériliser peut être totalement contenu dans le premier volume 10a. Les figures 2 à 5 illustrent quelques modes de réalisation en liaison avec deux formes particulières, sans que l'invention doive être limitée à ces deux formes.

L'emballage de stérilisation 1 est employé lors d'un procédé de stérilisation illustré par la figure 6.

Lors d'une première étape 21, un objet à stériliser est introduit dans un emballage par l'intermédiaire du quatrième bord, ouvert, de sorte à être totalement compris dans le premier volume 10a de l'emballage. On rappelle que le premier volume 10a est défini comme correspondant au volume de l'emballage en regard de la première partie 2 de la face avant. Le quatrième bord est ensuite refermé, notamment par soudure thermique. En effet, le matériau poreux est poreux aux gaz mais pas aux molécules plus grosses, tel que les bactéries et virus. Le quatrième bord fermé et le matériau poreux permettent de préserver la stérilisation de l'objet en sortie de l'autoclave tout en laissant passer l'air et la vapeur d'eau.

Lors d'une deuxième étape 22, l'emballage de stérilisation 1 comprenant l'objet à stériliser est introduite dans un autoclave. La stérilisation est alors déclenchée, notamment par cyclage de vapeur d'eau chauffée à 125°C et de vide. Du fait de la présence d'au moins une partie en matériau poreux dans l'emballage, l'objet à stériliser compris dans l'emballage est également soumis à la vapeur d'eau. A la fin du cycle de stérilisation, l'emballage et l'objet sont séchés par cyclage de vide et d'air.

Lors d'une troisième étape 23, l'emballage de stérilisation 1 comprenant l'objet à stériliser est retiré de l'autoclave et scellé. Afin d'être scellé, une soudure thermique des parties plastiques opposées est réalisée à la jonction entre la première partie et la deuxième partie de la face avant de sorte que le premier volume soit scellé avec l'objet stérilisé à l'intérieur. Le deuxième volume 10b comprenant la au moins une partie en matériau poreux est alors éliminé après découpe. La soudure thermique est réalisée par l'intermédiaire d'une double barre chauffée ou d'un double rouleau chauffé appliqués des deux côtés à la zone à souder de façon connue de l'état de la technique.

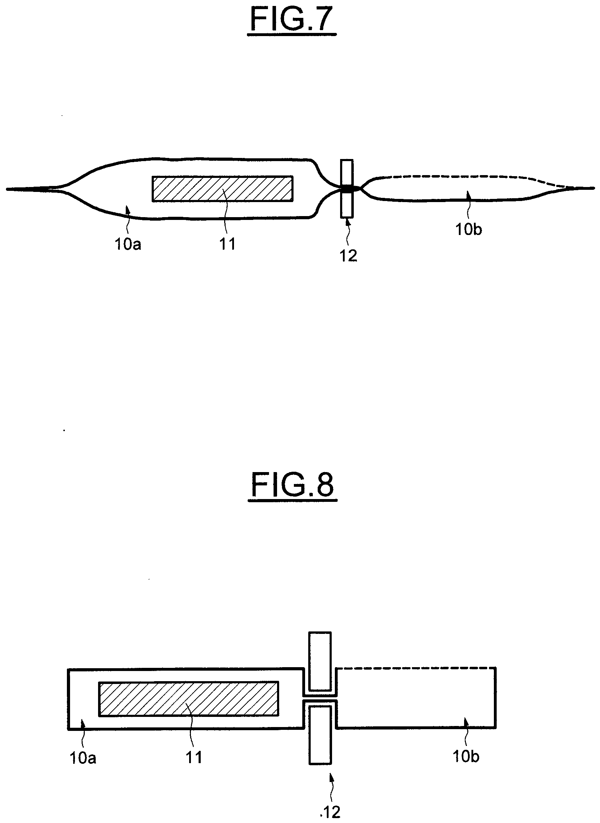

La figure 7 illustre le résultat du procédé de scellage de l'emballage de stérilisation selon le premier mode de réalisation, avant découpe et élimination du deuxième volume 10b. La figure 8 illustre le résultat du procédé de scellage de l'emballage de stérilisation selon le deuxième mode de réalisation. Dans les deux cas, un objet à stériliser, référencé 11, est disposé dans le premier volume 10a de l'emballage de stérilisation. Un dispositif de soudure thermique, référencé 12 a été appliqué de part et d'autre de l'emballage entre le premier volume 10a et le deuxième volume 10b, de sorte que la premier volume 10a est hermétiquement scellé avec l'objet 11 stérilisé à l'intérieur. Le deuxième volume 10b peut ensuite être séparé du premier volume 10a, notamment par découpe. Avantageusement, la soudure et la découpe sont réalisées simultanément par le même dispositif.

Ainsi scellé dans le premier volume 10a de l'emballage en matériau étanche, l'objet stérilisé peut être admis dans une salle blanche par l'intermédiaire d'un sas de transfert, impliquant la mise en contact avec la solution aqueuse de peroxyde d'hydrogène. Cependant, le premier volume 10a de l'emballage étant scellé et étanche, le peroxyde d'hydrogène ne peut entrer en contact avec l'objet stérilisé, et, dans le cas d'un objet perméable ou imprégnable, imbiber ledit objet stérilisé, provoquant le blocage du sas de transfert.

## Revendications

1. Enveloppe de stérilisation destinée à recevoir un objet à stériliser, comprenant une face avant et une face arrière, jointes sur leur périphérie sur trois bords, **caractérisé par le fait que** la face avant comprend une première partie (2) réalisée en matériau étanche et une deuxième partie (3) réalisée en matériau poreux, de sorte que le volume de l'emballage en regard de la première partie (2) de la face avant puisse être scellé à la jonction entre la première partie (2) et la deuxième partie (2) après stérilisation afin d'être étanche.

2. Enveloppe de stérilisation selon la revendication 1, dans lequel le matériau étanche est une matière plastique souple, notamment du Polyphénylène éther, du polypropylène, du polyester ou tout autre matériau plastique souple, le matériau poreux étant notamment du papier.

3. Enveloppe de stérilisation selon l'une quelconque des revendications 1 ou 2, dans laquelle la face arrière comprend une unique partie en matériau étanche.

4. Enveloppe de stérilisation selon l'une quelconque des revendications 1 à 3, comprenant des faces périphériques de sorte à former un parallélépipède, les faces périphériques (4) étant réalisées en matériau étanche.

5. Enveloppe de stérilisation selon l'une quelconque des revendications 1 ou 2, dans laquelle une première partie (6) de la face arrière est en matériau étanche, une deuxième partie (8) de la face arrière étant en matériau poreux.

6. Enveloppe de stérilisation selon la revendication 5, comprenant des faces périphériques de sorte à former un parallélépipède, les faces périphériques ou parties des faces périphériques (9) en regard des premières parties (2,6), étant réalisées en matériau étanche, les faces périphériques ou parties des faces périphériques (7) en regard des deuxièmes parties (3,8), étant réalisées en matériau poreux.

7. Procédé de stérilisation d'un objet comprenant les étapes suivantes :
- Introduction de l'objet à stériliser dans un emballage tel que revendiqué dans l'une quelconque des revendications 1 à 6 par l'intermédiaire du quatrième bord ouvert, l'objet étant disposé de sorte à être totalement compris dans un premier volume (10a) de l'emballage correspondant au volume de l'emballage en regard de la première partie (2) de la face avant, et fermeture du quatrième bord ouvert,
- Stérilisation de l'emballage de stérilisation (1) comprenant l'objet à stériliser dans un autoclave, l'objet à stériliser étant stérilisé du fait de la présence d'au moins une partie en matériau poreux dans l'emballage, permettant les échanges gazeux, notamment de vapeur, avec l'atmosphère à l'intérieur de l'autoclave,
- Retrait de l'emballage de stérilisation (1) comprenant l'objet stérilisé de l'autoclave,
- Scellement de l'emballage de stérilisation (1) comprenant l'objet stérilisé par soudure thermique à la jonction entre la première partie et la deuxième partie de la face avant de sorte que l'objet stérilisé à l'intérieur soit scellé dans le premier volume (10a) de l'emballage entièrement en matériau étanche, et
- Découpe et élimination du deuxième volume (10b) de l'emballage de stérilisation.
